# EUROPEAN PATENT SPECIFICATION

(11) **EP 4 021 385 B1**
(45) Date of publication and mention of the grant of the patent: **04.02.2026**
(21) Application number: 20842916.7
(22) Date of filing: 18.12.2020
(51) Int. Cl.: A61K 8/19, A61K 8/24, A61K 8/27, A61K 8/34, A61Q 11/00

(54) **ORAL CARE COMPOSITIONS AND METHODS OF USE**
MUNDPFLEGEZUSAMMENSETZUNGEN UND VERFAHREN ZUR VERWENDUNG
COMPOSITIONS DE SOINS BUCCO-DENTAIRES ET PROCÉDÉS D'UTILISATION

(30) Priority: 20.12.2019 US 201962951592 P
(43) Date of publication of application: 06.07.2022
(73) Proprietor: Colgate-Palmolive Company, New York, NY 10022 (US)
(72) Inventor: THOMSON, Paul, Piscatatway, New Jersey 08854 (US); D'AMBROGIO, Robert, Princeton, New Jersey 8540 (US); DENIS, Jean, Union, New Jersey 07083 (US); XU, Guofeng, Plainsboro, New Jersey 8536 (US)
(74) Representative: Sonnenhauser, Thomas Martin
(86) International application number: PCT/US2020/070926
(87) International publication number: WO 2021/127699

(56) References cited:
- WO-A1-91/07163
- US-A1- 2017 367 939

## Description

This invention relates to oral care compositions comprising a first stannous ion source, a second stannous ion source, wherein the second stannous ion comprises stannous pyrophosphate, and a source of zinc selected from zinc oxide, zinc citrate, zinc lactate, and combinations thereof, and a humectant system comprising glycerin and sorbitol, and a thickening system, as well as to methods of using and making these compositions.

### BACKGROUND

Oral care compositions present particular challenges in preventing microbial contamination.

Stannous ions, in particular stannous salts such as stannous fluoride, are known anti-microbial agents and are used in various dentifrices as agents for preventing plaque. However, there are certain disadvantages to using stannous salts, such as instability, tendency to stain teeth, astringency, and unpleasant taste for users.

Zinc is also a known antimicrobial agent used in toothpaste compositions. Zinc is a known essential mineral for human health, and has been reported to help strengthen dental enamel and to promote cell repair. Unfortunately, conventional toothpaste formulations often require high concentrations of zinc, e.g., 2% by weight or more, to achieve efficacy. At this concentration, the zinc imparts a notably astringent taste to the composition. There is thus a need for improved antibacterial toothpaste formulations that do not suffer from the drawbacks of conventional compositions.

However, due to formula complexity of certain oral care compositions which contain both stannous fluoride and one or more zinc sources, these formulas can be a challenge to produce from both a manufacturing and supply chain perspective.

Accordingly, in view of the drawbacks and disadvantages to using various antimicrobials, such as zinc and stannous, there is a need for oral care compositions with anti-bacterial efficacy, but which are also palatable and desirable for a user.
WO 91/07163 A1 discloses an aqueous stannous fluoride composition for oral care, containing stannous fluoride and lower alkyl vinyl ether and maleic anhydride or maleic acid copolymer in an amount sufficient to efficiently stabilize the stannous fluoride concentration, the composition being substantially free from soluble pyrophosphates, silica containing compounds and aldehyde containing flavoring agents.
US 2017/367939 A1 discloses an oral care compositions comprising a first source of stannous, a second source of stannous, wherein the second source of stannous contains stannous pyrophosphate, and a zinc source selected from the group consisting of: zinc oxide, zinc citrate, zinc lactate, and combinations thereof, as well as to methods of using and of making these compositions.

### BRIEF SUMMARY

Certain oral care compositions comprising stannous fluoride and zinc use glycerin as the main or primary humectant. However, it has been surprisingly found that, in those same zinc and stannous fluoride compositions, sorbitol can actually be used to supplement or partially replace glycerin. The addition of sorbitol, at certain percentages and weights, affords easier processing of the oral care composition but still allows the oral care formulations to maintain or even improve therapeutic performance in certain assays, as well as chemical and physical stability. Without being bound by theory, the addition of sorbitol at defined concentrations may allow for reduced heating and mixing time for processing for certain formula structuring agents, for example, hydroxyethylcellulose (HEC).

Furthermore, stannous fluoride formulations that contain zinc oxide and zinc citrate can provide much needed antibacterial benefits. However, another unexpected discovery is that stannous fluoride formulas with both sorbitol and glycerin, and which contain stannous pyrophosphate but only a single zinc source (i.e., zinc oxide), can provide enhanced antibacterial performance relative similar formulas that comprise both zinc oxide and zinc citrate.

The invention is an oral care composition comprising:
a. a zinc source, wherein the zinc source comprises zinc oxide and zinc citrate;
b. a first source of stannous (e.g., stannous fluoride);
c. a second source of stannous, wherein the second source of stannous comprises stannous pyrophosphate;
d. a humectant system comprising glycerin and sorbitol, and wherein the sorbitol is in an amount of from 2.5to 35% by wt.% (e.g., about 3 wt.%, about 3.5 wt.%, about 5 wt.%, about 7 wt.%, about 10 wt.%, about 10.5 wt.%, about 14 wt.%, about 15 wt.%, about 20 wt.%, about 21 wt.%, about 24 wt.%, about 25 wt.%, about 30 wt.%) (e.g., 5 to 35 wt.%); and
e. a thickening system comprising a thickening agent selected from the group consisting of carrageenan, hydroxyethyl cellulose, and water-soluble salts of cellulose ethers (e.g., sodium carboxymethyl cellulose and sodium carboxymethyl hydroxyethyl cellulose), and combinations thereof, wherein the wt.% is based on the total weight of the oral care composition.

Unless otherwise indicated, values are given as percentage of the overall weight of the composition.

The zinc source comprises zinc oxide and zinc citrate (e.g., zinc trihydrate).

The amount of zinc oxide (e.g., wt.%) to zinc citrate (e.g., wt%) may be from 1.5:1 to 4.5:1 (e.g., 2:1, 2.5:1, 3:1, 3.5:1, or 4:1).

The zinc citrate may be present in an amount of from 0.25 to 1.0 wt% (e.g., 0.5 wt. %) and zinc oxide may be present in an amount of from 0.75 to 1.25 wt% (e.g., 1.0 wt. %) based on the weight of the oral care composition.

The zinc citrate (e.g., zinc trihydrate) may be present in an amount of about 0.5 wt%. The zinc oxide may be present in an amount of about 1.0 wt%.

The zinc citrate (e.g., zinc trihydrate) may be present in an amount of about 0.5 wt% and the zinc oxide may be present in an amount of about 1.0 wt%.

The zinc oxide may be present in an amount of about 1.0 wt%.

The zinc citrate may be present in an amount of about 0.8 wt% (e.g., about 0.85 wt.%) and the zinc oxide may be present in an amount of about 1.0 wt%.

The amount of stannous pyrophosphate may be from 0.1% - 3% by wt. of the composition. (e.g., about 1% by wt. of the composition).

The first stannous ion source may be stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof.

The first stannous ion source may be stannous fluoride (e.g., about 0.45 wt%; e.g., about 0.454 wt%.)

The composition may further comprise a fluoride source selected from: stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.

The pH may be between 7.5 and 10.5.

The composition may further comprise an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures of any of two or more of these, e.g., in an amount of 1-20%, e.g., 2-8%, e.g., ca. 5%>, by weight of the composition.

The alkali phosphate salt may comprise tetrapotassium pyrophosphate;
preferably the tetrasodium pyrophosphate may be present in an amount from .1 - 3.0 wt% (e.g., about 2.0 wt%);
more preferably the salt comprises sodium tripolyphosphate;
even more preferably sodium tripolyphosphate is from 0.1 - 3.0 wt% (e.g., about 2.0 wt%).

The composition may further comprise an abrasive or particulate (e.g., silica).

The silica may be synthetic amorphous silica. (e.g., 1% - 28% by wt.) (e.g., 8% - 25% by wt.).

The silica abrasives may be silica gels or precipitated amorphous silicas, e.g. silicas having an average particle size ranging from 2.5 microns to 12 microns.

The composition may further comprise a small particle silica having a median particle size (d50) of 1- 5 microns (e.g., 3 - 4 microns) (e.g., about 5 wt. % Sorbosil AC43 from PQ Corporation, Warrington, United Kingdom).

20-30 wt% of the total silica in the composition may be small particle silica (e.g., having a median particle size (d50) of 3 -4 microns), wherein the small particle silica is present in an amount of about 5 wt.% of the oral care composition.

The glycerin may be present in a total amount of 30- 45% (e.g., about 42%).

The glycerin may be present in an amount of about 42% by wt. of the composition.

The composition may comprise an aqueous buffer system, for example, wherein the buffer system comprises an organic acid and an alkali metal salt thereof, e.g., wherein the organic acid is citric acid and the salt is a mono-, di- and/or tri- alkali metal citrate salt, e.g., mono-, di- and/or tri- lithium, sodium, potassium, or cesium citrate salt, and citric acid.).

The buffer system may comprise tri-sodium citrate and citric acid (e.g., 1 to 10% by weight of the composition) (e.g., 2.1% by wt. of the composition). For example, the molar ratio of mono-, di- and/or tri-sodium citrate and citric acid is 1.5 to 5, (e.g., 2 to 4). The buffer may be a citrate buffer comprising sodium citrate (e.g., about 1.5% wt.) and citric acid (e.g., about 0.6% wt.)

The composition may comprise polymer films.

The composition may comprise flavoring, fragrance and/or coloring.

The thickening system may comprise hydroxyethyl cellulose (e.g., hydroxyethyl cellulose and carrageenan) (e.g., from 0.2% - 0.75% total wt% of hydroxyethyl cellulose and carrageenan).

The thickening system may comprise sodium carboxymethyl cellulose (e.g., from 0.5 wt.% - 1.5 wt.%).

The composition may comprise from 5% - 40%, e.g., 10% - 35%, e.g., about 10%, 15%, 25%, 30%, and 35% water.

The composition may comprise an additional antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, Zinc Chloride, Zinc Lactate, Zinc Sulfate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.

The composition may comprise an antioxidant, e.g., selected from the group consisting of Co-enzyme Q10, PQQ, Vitamin C, Vitamin E, Vitamin A, BHT, anethole-dithiothione, and mixtures thereof.

The composition may comprise a whitening agent.

The composition may comprise a whitening agent selected from a whitening active selected from the group consisting of peroxides, metal chlorites, perborates, percarbonates, peroxyacids, hypochlorites, and combinations thereof.

The whitening agent may be titanium dioxide.

The composition may further comprise hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.

The composition may further comprise a polymer, e.g., an anionic polymer, for example a polycarboxylate polymer (e.g., PVM/MA copolymer, in an amount of from 0.1-5%, e.g., 0.2-2%, e.g., 0.3-1%.

The composition may further comprise microcrystalline cellulose and/or sodium carboxymethylcellulose, e.g., in an amount of from 0.1-5%, e.g., 0.5-2%, e.g. 1%.

The composition may further comprise one or both of:
a. Polyethylene glycol in an amount of from 1-6% (e.g., 3% wt.); and
b. Propylene glycol in an amount of from 1-6% (e.g., 4% wt.).

The composition may further comprise polyvinylpyrrolidone (PVP) in an amount of from 0.5-3 wt. %, e.g. about 1.25 wt. %.

The composition may further comprise an agent that interferes with or prevents bacterial attachment, e.g., ELA or chitosan.

The composition may comprise glycerin in an amount from 20% - 45% by wt. of the composition (e.g., about 20% by wt., e.g., about 22% by wt., e.g., about 25% by wt, e.g., about 30%, e.g., about 35%, e.g., about 40%, e.g., about 43%)

The humectant system may comprise glycerin and sorbitol in a wt% ratio (glycerin:sorbitol) from 1:0.10 to 0.75:1, wherein the wt% is by weight of the humectant system.

Sorbitol may be present in an amount from 2.5 to 30 wt.%, wherein the wt.% is based on the total weight of the oral care composition(e.g., about 3.5 wt.%, about 7 wt.%, about 15 wt.%, about 17 wt.%, about 20 wt.%, about 24 wt.%, about 24.5 wt.%, about 25 wt.%, about 30 wt.%)

Sorbitol may be present in an amount from 5to 35wt.%, wherein the wt.% is based on the total weight of the oral care composition (e.g., about 5 wt.%, about 10 wt.%, about 15 wt.%, about 17 wt.%, about 20 wt.%, about 25 wt.%, about 30 wt.%).

Sorbitol may be present in an amount from 2.5 to 25wt.%, wherein the wt.% is based on the total weight of the oral care composition (e.g., about 3 wt.%, e.g., about 3.5 wt.%, e.g., about 5 wt.%, e.g., about 17 wt.%, e.g., about 20 wt.%, e.g., about 21 wt.%, e.g., about 24 wt.%, e.g., about 25 wt.%).

Sorbitol may be present in an amount from 5to 25 wt.%, wherein the wt.% is based on the total weight of the oral care composition.

Sorbitol may be present in an amount from 20 to 30 wt.%, wherein the wt.% is based on the total weight of the oral care composition.

Sorbitol may be present in an amount from 25% - 35 wt.%, wherein the wt.% is based on the total weight of the oral care composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.%
e. a humectant system comprising glycerin and sorbitol, wherein the sorbitol is present in an amount of about 3.5 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.%
e. a humectant system comprising glycerin and sorbitol, wherein the sorbitol is present in an amount of about 5 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.%
e. a humectant system comprising glycerin and sorbitol, wherein the sorbitol is present in an amount of about 7 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.%
e. a humectant system comprising glycerin and sorbitol, wherein the sorbitol is present in an amount of about 10 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 14 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 15 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is in an amount of about 17 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 20 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 21 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 24.5 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.% and
e. a humectant system comprising glycerin and sorbitol, wherein the sorbitol is present in an amount of about 25 wt.%; and
f. a thickening system comprising hydroxyethyl cellulose and carrageenan, wherein the wt.% is based on the total weight of the composition.

The composition may comprise:
a. Zinc oxide in an amount of about 1.0 wt.%
b. Zinc citrate in an amount of about 0.5 wt.%
c. Stannous fluoride (e.g., in an amount of about 0.45 wt.%)
d. Stannous pyrophosphate in an amount of about 1.0 wt.%;
e. a humectant system comprising glycerin and sorbitol, wherein the sorbitol is present in an amount of about 30 wt.%; and
f. a thickening system comprising hydroxyethyl cellulose and carrageenan, wherein the wt.% is based on the total weight of the composition.

The Composition may further comprise a citrate buffer system, wherein the buffer system comprises tri-sodium citrate and citric acid (e.g., the buffer system being about 2.1 wt% of the composition).

The composition may be effective upon application to the oral cavity, e.g., by rinsing, optionally in conjunction with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) clean the teeth and oral cavity (xiv) reduce erosion, (xv) prevents stains and/or whiten teeth, (xvi) immunize the teeth against cariogenic bacteria; and/or (xvii) promote systemic health, including cardiovascular health, e.g., by reducing potential for systemic infection via the oral tissues.

The oral composition may be any of the following oral compositions selected from the group consisting of: a toothpaste or a dentifrice, a mouthwash or a mouth rinse, a topical oral gel, and a denture cleanser.

A composition may be obtained or obtainable by combining its ingredients.

Zinc oxide and zinc citrate may be the only sources of zinc.

Stannous fluoride and stannous pyrophosphate may be the only sources of stannous. The composition may be a dentifrice or a mouthwash.

The composition may be any of the following selected from: a toothpaste, transparent paste, gel, mouth rinse, spray and chewing gum.

The composition may be incorporated into a chewing gum.

A composition may be obtained or obtainable by combining its ingredients.

The invention also encompasses the oral composition of the invention for use in a method to improve oral health comprising applying an effective amount of the oral composition of any of the embodiments set forth above to the oral cavity of a subject in need thereof, e.g., to
i. reduce or inhibit formation of dental caries,
ii. reduce, repair or inhibit early enamel lesions, e.g., as detected by quantitative light- induced fluorescence (QLF) or electrical caries measurement(ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial bio film formation in the oral cavity,
x. raise and/or maintain plaque pH at levels of at least pH 5.5 following sugar challenge,
xi. reduce plaque accumulation,
xii. treat dry mouth,
xiii. enhance systemic health, including cardiovascular health,e.g., by reducing potential for systemic infection via the oral tissues,
xiv. Whiten teeth,
xv. reduce erosion of the teeth,
xvi.immunize (or protect) the teeth against cariogenic bacteria and their effects, and/or
xvii. clean the teeth and oral cavity.

Sodium bicarbonate, sodium methyl cocoyl taurate (tauranol), MIT, and benzyl alcohol and combinations thereof may be used in the manufacture of the Composition of the Invention, e.g., for use in a method to improve oral health comprising applying an effective amount of said oral composition, wherein the oral care composition is applied to the oral cavity of a subject in need thereof, in any of the indications set forth above.

### DETAILED DESCRIPTION

As used herein, the term "oral composition" means the total composition that is delivered to the oral surfaces. The composition is further defined as a product which, during the normal course of usage, is not, the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

### Stannous Ion Source

In some embodiments, the first stannous source comprises a stannous source selected from stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or mixtures thereof. In some embodiments, the first stannous source comprises stannous fluoride.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources used with the present invention include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

### Surfactants

The Composition of the invention may in some embodiments contain anionic surfactants, e.g., water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOS0₃X, wherein m is 6-16, e.g., 10, n is 1-6, e.g., 2, 3 or 4, and X is Na or , for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆₋₃o alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants, that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In another embodiment illustrative zwitterionic surfactants, that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent may be incorporated in the oral composition at a concentration of 0.01 to 1% by weight.

### Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts that may be used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition may be generally enough to provide at least 0.1 wt. % pyrophosphate ions, e.g., 0.1 to 3 wt 5, e.g., 0.1 to 2 wt %, e.g., 0.1 to 1 wt%, e.g., 0.2 to 0.5 wt%. The pyrophosphates also contribute to preservation of the compositions by lowering water activity.

In various embodiments of the present disclosure, the compositions further comprise one or more anticalculus (tartar control) agents. Suitable anticalculus agents include without limitation mono-phosphates (e.g. monobasic, dibasic or tribasic phosphate) and P1-6 polyphosphates (e.g., pyrophosphates, tripolyphosphate, tetraphosphates and hexametaphosphate salts, zinc salts (e.g., zinc citrate, zinc chloride, zinc citrate trihydrate), Gantrez^{®} (a copolymer of methylvinyl ether (PVM) and maleic acid (MA)), polyaminopropanesulfonic acid (AMPS), polypeptides, polyolefin sulfonates, polyolefin phosphates, and diphosphonates. In certain embodiments, the other anticalculus agents are alkali and/or alkaline earth metal phosphate salts, for example, sodium, potassium or calcium salts. In certain embodiments, the composition includes mono-phosphates (e.g. monobasic, dibasic or tribasic phosphate), P1-6 polyphosphates, Gantrez, or a combination thereof. Still in certain embodiments, the composition includes sodium tripolyphosphate, tetrasodium pyrophosphate, Gantrez, or a combination thereof.

### Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

In preparing oral care compositions, it is sometimes necessary to add some thickening material to provide a desirable consistency or to stabilize or enhance the performance of the formulation. The thickening agents are selected from the group consisting of carrageenan, hydroxyethyl cellulose and water soluble salts of cellulose ethers such as sodium carboxymethyl cellulose and sodium carboxymethyl hydroxyethyl cellulose. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Abrasives

Natural calcium carbonate is found in rocks such as chalk, limestone, marble and travertine. It is also the principle component of egg shells and the shells of mollusks. The natural calcium carbonate abrasive of the invention is typically a finely ground limestone which may optionally be refined or partially refined to remove impurities. For use in the present invention, the material has an average particle size of less than 10 microns, e.g., 3-7 microns, e.g. about 5.5 microns. For example a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ^{®} 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. For use in the present invention, the particles are small, e.g., having an average particle size of 1 - 5 microns, and e.g., no more than 0.1 %, preferably no more than 0.05% by weight of particles which would not pass through a 325 mesh. The particles may for example have a D50 of 3-6 microns, for example 3.8=4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/100g, e.g. 30-70 g/100g. Examples of commercially available products suitable for use in the present invention include, for example, Carbolag^{®} 15 Plus from Lagos Industria Quimica.

In certain embodiments the invention may comprise additional calcium-containing abrasives, for example calcium phosphate abrasive, e.g., tricalcium phosphate (Ca₃(P0₄)₂), hydroxyapatite (Ca₁₀(P0₄)₆(OH)₂), or dicalcium phosphate dihydrate (CaHP0₄ · 2H₂0, also sometimes referred to herein as DiCal) or calcium pyrophosphate, and/or silica abrasives, sodium metaphosphate, potassium metaphosphate, aluminum silicate, calcined alumina, bentonite or other siliceous materials, or combinations thereof. Any silica suitable for oral care compositions may be used, such as precipitated silicas or silica gels. For example synthetic amorphous silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

### Water

Water may be present in the oral compositions of the invention. Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

### Humectants

A humectant system is incorporated in the composition of the invention to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerine, sorbitol, xylitol, propylene glycol as well as other polyols and mixtures of these humectants. Glycerine and sorbitol are comprised by the humectant system of the compositions if the invention; wherein the sorbitol is present in an amount of from 2.5 to 35 wt.%, wherein the wt.% is based on the total weight of the oral care composition.

The composition of the present invention for use in a method involves applying to the oral cavity an effective amount of the compositions described herein.

The compositionaccording to the invention can be any of the following selected from: toothpastes, transparent pastes, gels, mouth rinses, sprays and chewing gum.

As used throughout, ranges are used as shorthand for describing each and every value that is within the range. Any value within the range can be selected as the terminus of the range. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the scope thereof.

### Example 1

**Table 1.**

| **REPRESENTATIVE TOOTHPASTE FORMULATIONS** | | | |
|---|---|---|---|
| | **Formula A** | **Formula B** | **Formula C** |
| Material Description | Wt% | Wt% | Wt% |
| GLYCERIN - USP, EP VEG | 43.2 | 38.5 | 33.5 |
| ABRASIVE SILICA | 19.0 | 19.0 | 19.0 |
| SORBITOL - NON-CRYSTAL - 70% SOLN USP, EP | | 5.0 | 10.0 |
| DEMINERALIZED WATER | Q.S. | Q.S. | Q.S. |
| AMORPHOUS SILICA | 5.0 | 5.0 | 5.0 |
| HUMECTANT OTHER THAN GLYCERIN OR SORBITOL | 4.0 | 4.0 | 4.0 |
| POLYMERS | 4.25 | 4.0 | 4.0 |
| ALKALI PHOSPHATE SALTS | 3.0 | 3.0 | 3.0 |
| ANIONIC SURFACTANT | 1.75 | 1.75 | 1.75 |
| FLAVORS, COLORS, SWEETENERS | 2.25 | 2.25 | 2.25 |
| STANNOUS PYROPHOSPHATE | 1.0 | 1.0 | 1.0 |
| ZWITTERIONIC SURFACTANT | 1.0 | 1.0 | 1.0 |
| Microcrystalline Cellulose/Sodium CMC NF | 1.0 | 1.0 | 1.0 |
| ZINC OXIDE | 1.0 | 1.0 | 1.0 |
| COPOLYMER OF METHYLVINYL ETHER (PVM) AND MALEIC ACID (MA) | 0.606 | 0.606 | 0.606 |
| CITRATE BUFFER | 2.1 | 2.1 | 2.1 |
| 85% SYRUPY PHOSPHORIC ACID - FOOD GRADE | 0.55 | 0.55 | 0.55 |
| ZINC CITRATE TRIHYDRATE | 0.5 | 0.5 | 0.5 |
| STANNOUS FLUORIDE, USP | 0.454 | 0.454 | 0.454 |
| HYDROXYETHYLCELLULOS E | 0.3 | 0.25 | 0.25 |
| CARRAGEENAN CONCENTRATE | 0.3 | 0.25 | 0.25 |
| Total Components | 100.0 | 100.0 | 100.0 |
| Total Water | ~ 9.3 | 10.8 | 12.3 |

**Table 1. (continued)**

| | **Formul aD** | **Formul aE** | **Formul aF** | **Formula G** |
|---|---|---|---|---|
| Material Description | Wt% | Wt% | Wt% | Wt% |
| GLYCERIN - USP, EP VEG | 28.5 | 23.5 | 22.5 | 22.6 |
| ABRASIVE SILICA | 19.0 | 19.0 | 20.0 | 20.0 |
| SORBITOL - NON-CRYSTAL - 70% SOLN USP, EP | 15.0 | 20.0 | 30.0 | 30.0 |
| DEMINERALIZED WATER | Q.S. | Q.S. | Q.S. | Q.S. |
| AMORPHOUS SILICA | 5.0 | 5.0 | | |
| HUMECTANT OTHER THAN GLYCERIN OR SORBITOL | 4.0 | 4.0 | | |
| POLYMERS | 4.0 | 4.0 | 4.0 | 4.0 |
| ALKALI PHOSPHATE SALTS | 3.0 | 3.0 | 3.0 | 3.0 |
| ANIONIC SURFACTANT | 1.75 | 1.75 | 1.20 | 1.20 |
| FLAVORS, COLORS, SWEETENERS | 2.25 | 2.25 | 1.45 | 1.45 |
| STANNOUS PYROPHOSPHATE | 1.0 | 1.0 | 1.000 | 1.0 |
| ZWITTERIONIC SURFACTANT | 1.0 | 1.0 | 0.60 | 0.60 |
| Microcrystalline Cellulose/Sodium CMC NF | 1.0 | 1.0 | 1.0 | 1.0 |
| ZINC OXIDE | 1.0 | 1.0 | 1.0 | 1.0 |
| COPOLYMER OF METHYLVINYL ETHER (PVM) AND MALEIC ACID (MA) | 0.606 | 0.606 | | |
| CITRATE BUFFER | 2.1 | 2.1 | 2.1 | 2.1 |
| 85% SYRUPY PHOSPHORIC ACID - FOOD GRADE | 0.55 | 0.55 | 0.55 | 0.55 |
| ZINC CITRATE TRIHYDRATE | 0.5 | 0.5 | 0.5 | |
| STANNOUS FLUORIDE, USP | 0.454 | 0.454 | 0.454 | 0.454 |
| HYDROXYETHYLCELLULOSE | 0.25 | 0.25 | 0.3 | 0.3 |
| CARRAGEENAN CONCENTRATE | 0.25 | 0.25 | 0.3 | 0.3 |
| THICKENING SILICA | | | 1.25 | 1.50 |
| Total Components | 100.0 | 100.0 | 100.0 | 100.0 |
| Total Water | 13.8 | 15.3 | 18.1 | 18.3 |

### Example 2

*In vitro* assay for monitoring metal ion deposition

**Table 2.**

| | | **A** | **B** | **C** | **D** | **E** | **F** | **G** |
|---|---|---|---|---|---|---|---|---|
| pH (10% Soln) | Initial | 7.10 | 6.87 | 6.77 | 6.86 | 7.28 | 6.96 | 7.18 |
| | 4wks-40C/75%RH | 6.73 | 7.16 | 7.01 | 6.66 | 7.26 | 6.99 | 7.27 |
| | 8wks-40C/75%RH | 6.89 | 7.12 | 7.17 | 7.16 | 7.22 | 7.09 | 7.22 |
| | 13wks-30C/65%RH | 6.94 | 7.22 | 7.08 | 6.95 | 7.10 | 7.10 | 7.29 |
| | 13wks-40C/75%RH | 6.95 | 7.23 | 7.07 | 7.10 | 7.14 | 7.16 | 7.33 |
| | | | | | | | | |
| Sol. Fluoride (ppm) | Initial | 112 8 | 103 6 | 104 2 | 106 5 | 107 5 | 108 8 | 104 8 |
| | 4wks-40C/75%RH | 950 | 931 | 901 | 889 | 896 | 100 2 | 916 |
| | 8wks-40C/75%RH | 869 | 865 | 101 9 | 803 | 838 | 987 | 867 |
| | 13wks-30C/65%RH | 100 9 | 764 | 899 | 840 | 910 | 893 | 876 |
| | 13wks-40C/75%RH | 791 | 692 | 713 | 688 | 719 | 705 | 711 |
| | | | | | | | | |
| Total Tin (%) | Initial | 0.80 | 0.83 | 0.83 | 0.86 | 0.90 | 0.83 | 0.81 |
| | | | | | | | | |
| Sol. Tin (%) | Initial | 0.43 | 0.54 | 0.45 | 0.60 | 0.55 | 0.64 | 0.45 |
| | 4wks-40C/75%RH | 0.40 | 0.47 | 0.55 | 0.52 | 0.53 | 0.65 | 0.49 |
| | 8wks-40C/75%RH | 0.39 | 0.43 | 0.47 | 0.50 | 0.50 | 0.64 | 0.48 |
| | 13wks-30C/65%RH | 0.43 | 0.47 | 0.43 | 0.58 | 0.52 | 0.66 | 0.42 |
| | 13wks-40C/75%RH | 0.38 | 0.41 | 0.46 | 0.51 | 0.39 | 0.59 | 0.40 |
| | | | | | | | | |
| Total Zinc (%) | Initial | 0.92 | 0.97 | 0.96 | 0.95 | 0.89 | 1.00 | 0.72 |
| | | | | | | | | |
| Sol. Zinc (%) | Initial | 0.65 | 0.60 | 0.59 | 0.59 | 0.69 | 0.62 | 0.47 |
| | 4wks-40C/75%RH | 0.67 | 0.54 | 0.56 | 0.53 | 0.61 | 0.59 | 0.43 |
| | 8wks-40C/75%RH | 0.52 | 0.50 | 0.50 | 0.53 | 0.49 | 0.57 | 0.43 |
| | 13wks-30C/65%RH | 0.32 | 0.55 | 0.41 | 0.50 | 0.61 | 0.61 | 0.48 |
| | 13wks-40C/75%RH | 0.28 | 0.46 | 0.50 | 0.40 | 0.51 | 0.54 | 0.33 |
| | | | | | | | | |

Table 2 represents Stability summary of stannous fluoride toothpastes (described in Table 1).

Formulations in this invention can utilize up to 30% sorbitol to provide processing flexibility and, in some embodiments, may contain only a single zinc source. These formulas can contain up to about 18% total water due to contributions of sorbitol and other raw materials with some water content.

Formulas of Table 1 in Example 1 are evaluated for chemical and physical stability per ICH accelerated aging/stress guidelines and compared to a positive control formulation (Formula A) which does not contain any sorbitol. Based upon the data in Table 2, Formulas B-G of Table 1 are sufficiently stable for fluoride, soluble tin and soluble zinc and are acceptably buffered to maintain pH within 6.5-7.5 target range - See, Table 2.

The intention of these formulas is to provide a source of both soluble and insoluble metals within the toothpaste. Without being bound by theory, the insoluble sources may act as a reservoir that remain stable during shelf-life of the toothpaste but become available on dilution with saliva during brushing. The stability data of Table 2 demonstrates success in this practice, throughout the accelerated aging of the formulas, as both total tin and zinc are considerably greater percentages compared to soluble components. Also, the soluble components show relatively little change over the 13-week accelerated aging period which indicates successful metal stability, showing that compositions with some amount of sorbitol worked at parity to those compositions with only glycerin.

### Example 3

The University of Manchester anaerobic model is to provide a more sensitive indication of potential efficacy of the formulas described herein. In this model, saliva collected from 4 healthy volunteers is pooled together and used as inoculum. Each sample is treated in triplicate twice a day for 8 days. Biofilm is recovered after 16 treatments to measure for ATP (RLU) as an end point for viable bacteria. Toothpastes demonstrating lower ATP scores provide more effective antibacterial performance. Market based toothpaste formulations containing NaF and KNO3 actives are used as the "Negative Control" referred to in the tables below.

There are two separate tests in this study conducted with toothpaste prototypes of this invention. Formulas referred to here are the same as those described in Table 1, of Example 1, above.

In the first test, it is surprisingly found that both Formula B (containing 5% sorbitol, zinc citrate and zinc oxide) and Formula G (containing 30% sorbitol, only ZnO) perform statistically significantly better than the Formula A standard with no sorbitol. As can be seen, Formula G (30% sorbitol, single ZnO) provided superior performance over all other samples.

**Table 3. Viable Bacteria as ATP (RLU) - Manchester Model, Test 1**

| **Sample** | **Avg Log RLU** | **Statistical Comparison*** |
|---|---|---|
| Negative Control** | 4.652 +/- 0.213 | A |
| Formula A | 3.748 +/- 0.146 | B |
| Formula B | 3.370 +/- 0.120 | C |
| Formula G | 3.097 +/- 0.182 | D |

| | | |
|---|---|---|
| * Means that don't share common letter=Sign. Diff @95% CI, Tukey method, N=26 per cell ** Negative Control is the market-based formula containing NaF and KNO3 as the active ingredients. | | |

Both Formula A standard (Positive Control) and Formula G are included in an additional study and compared to Formula C (10% sorbitol, zinc oxide and zinc citrate). Formula G containing 30% sorbitol and zinc oxide (where zinc oxide is the only zinc source) performed very well, and at parity with the Positive Control (Formula) that has two zinc sources: zinc oxide and zinc citrate. All tested stannous fluoride formulations (Formulations A-G) performed significantly better than the negative control at controlling anaerobic biofilm.

**Table 4. Viable Bacteria as ATP (RLU) - Manchester Model, Test 2**

| **Sample** | **Avg Log RLU** | **Statistical Comparison*** |
|---|---|---|
| Negative Control** | 4.771 +/-0.130 | A |
| Formula A | 3.781 +/-0.122 | C,D |
| Formula C | 4.127 +/-0.177 | B |
| Formula G | 3.698 +/-0.153 | D |

| | | |
|---|---|---|
| * Means that don't share common letter=Sign. Diff @95% CI, Tukey method, N=26 per cell ** Negative Control is a market-based formula containing NaF and KNO3 as the active ingredients | | |

**Plaque glycolysis Model:** Formulas referred to here are the same as those described in Table 1 above.

An in vitro adaptation of a Glycolysis Model described by White, et. al., *Journal of Clinical Dentistry,* #6 Special Issue, p 69-78, (1995), the contents of which are incorporated herein by reference, is used to indirectly measure biofilm health. Briefly, the method quantifies the glycolytic effects of toothpaste formulas on treated in vitro biofilm pool of both anaerobic and aerobic bacteria. The efficacy of each toothpaste formula is based on biofilm pH change. A lower average pH change indicates reduction of viable bacteria and greater antibacterial performance of the respective test toothpaste. Finally, in these studies, an untreated cell is used as the negative control.

There are two separate plaque glycolysis tests conducted with toothpaste prototypes of this invention. In the first test, Formula A, Positive Control, is compared to Formula E (20% sorbitol, zinc citrate and zinc oxide). It is surprisingly found that the Formula E prototype is statically superior to the standard.

**Table 5. Plaque Glycolysis Study - Average pH Change with Treatment, Test 1**

| **Sample** | **Avg pH Change** | **Statistical Comparison*** |
|---|---|---|
| Untreated* * | 2.425 +/- 0.095 | A |
| Formula A | 0.761 +/- 0.023 | B |
| Formula E | 0.567 +/- 0.018 | C |

| | | |
|---|---|---|
| * Means that don't share common letter=Sign. Diff @95% CI, Tukey method, N=3 per cell ** Negative Control, untreated biofilm | | |

A second plaque glycolysis study compares Formula C (10% sorbitol, zinc citrate and zinc oxide) and Formula F (30% sorbitol, zinc oxide and zinc citrate) to the Positive Control (Formula A) and Negative Control (untreated biofilm). The 10% sorbitol formula of Formula C is comparable to the no sorbitol standard (Formula A). Whereas, the 30% sorbitol formula (i.e, Formula F) performs significantly better than the Formula A Positive Control standard.

**Table 6. Plaque Glycolysis Study - Average pH Change with Treatment, Test 2**

| **Sample** | **Avg pH Change** | **Statistical Comparison*** |
|---|---|---|
| Untreated** | 2.633 +/- 0.102 | A |
| Formula A | 0.856 +/- 0.069 | B |
| Formula C | 1.009 +/- 0.098 | B |
| Formula F | 0.549 +/- 0.045 | C |

| | | |
|---|---|---|
| * Means that don't share common letter=Sign. Diff @95% CI, Tukey method, N=3 per cell ** Negative Control, untreated biofilm | | |

### Example 4

The addition of sorbitol surprisingly helps in the manufacturing of the oral care compositions of the present invention.

In one aspect, the addition of sorbitol allows lowering the temperature at certain steps of the manufacturing process and to reduce the time taken to hydrate and mix the gums - e.g., HEC and Carrageenan - present in the formulation. In one aspect the addition of sorbitol enables lowering the temperature, and the time, to heat and hydrate the gums from 80 degrees Celsius (without sorbitol) to 50 degrees Celsius (with the addition of sorbitol).

Furthermore, Table 7 and Table 8 (below) also show that the physical stability of certain formulations containing 10% sorbitol, for example, function at parity, and/or demonstrate a slight improvement than formulations without sorbitol. The Formulas referred to in Tables 7 and 8 are the same as detailed in Table 1 (Example 1) above. Therefore, the addition of sorbitol in certain formulations can aid in manufacturing efficiency while retaining the physical stability of formulations without sorbitol:

**Table 7.**

| **Formula A:** | | | | | | |
|---|---|---|---|---|---|---|
| **1.0%SnPyro, 0.3%HEC, 0.3%Carr** | | | | | | |
| | **4wk-25C** | **4wk-40C** | **8wk-25C** | **8wk-40C** | **13wk-25C** | **13wk-40C** |
| Shear Stress (Pa) | 177 | 179 | 173 | 169 | 165 | 179 |
| Static Yield (Pa) | 56.9 | 70.2 | 65.1 | 87.2 | 68.9 | 68.2 |
| Dynamic Yield (Pa) | 15 | 15 | 15 | 15 | 14.2 | 14.5 |
| Viscosity @ 1 RPM (cP) | 332,360 | 312,393 | 291,781 | 356,836 | 289,205 | 356,836 |

**Table 8.**

| **Formula C: 1.0%SnPyro, 0.25%HEC 0.25%Carr, 10%Sorbitol** | | | | | | |
|---|---|---|---|---|---|---|
| | 4wk-25C | 4wk-40C | 8wk-25C | 8wk-40C | 13wk-25C | 13wk-40C |
| Shear Stress (Pa) | 180.72 | 190.10 | 201.87 | 194.18 | 265.315 | 344.14 |
| Static Yield (Pa) | 55.10 | 65.1 | 75.70 | 56.10 | 93.4 | 111.0 |
| Dynamic Yield (Pa) | 23.40 | 17.40 | 9.01 | 11.0 | 14.70 | 11.50 |
| Viscosity @ 1 RPM (cP) | 435,386 | 363,259 | 337,507 | 267,943 | 418,664 | 352,568 |

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

## Claims

1. An oral care composition comprising:
a. a zinc source, wherein the zinc source comprises zinc oxide and zinc citrate;
b. a first source of stannous;
c. a second source of stannous, wherein the second source of stannous comprises stannous pyrophosphate;
d. a humectant system comprising glycerin and sorbitol, and wherein the sorbitol is present in an amount of from 2.5 to 35 wt.%; and
e. a thickening system comprising a thickening agent selected from the group consisting of carrageenan, hydroxyethyl cellulose, and water-soluble salts of cellulose ethers, and combinations thereof,
wherein the wt.% is based on the total weight of the oral care composition.

2. The oral care composition of claim 1, wherein the ratio of the amount of zinc oxide to zinc citrate is from 1.5:1 to 4.5:1.

3. The oral care composition of any preceding claims, comprising zinc citrate and zinc oxide, wherein the zinc citrate is present in an amount of from 0.25 to 1.0 wt.%, and the zinc oxide may be present in an amount of from 0.75 to 1.25 wt.%, based on the total weight of the composition.

4. The oral care composition of any preceding claims, wherein the first stannous ion source is stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof.

5. The oral care composition of any preceding claims, wherein the first stannous ion source is stannous fluoride.

6. The oral care composition of any preceding claims, wherein the composition comprises one or more alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures thereof; preferably wherein the composition comprises an alkali phosphate salt in an amount of from1 to 20 wt.%, based on the total weight of the composition.

7. The oral care composition of any of the preceding claims, wherein the humectant system comprises glycerin and sorbitol in a wt.% ratio (glycerin:sorbitol) from 1:0.10 to 0.75:1, based on the weight of the humectant system.

8. The oral care composition of any of the preceding claims, wherein the sorbitol is present in an amount of from 2.5 to 25 wt.% based on the total weight of the composition.

9. The oral care composition of any of claims 1-8, wherein the sorbitol is present in an amount of from 5 to 35 wt.%, preferably in an amount of from 5 to 25 wt.%; or in an amount of from 25 to 35 wt.%, based on the total weight of the composition.

10. A composition of any of the preceding claims, wherein the composition comprises:
a. zinc oxide in an amount of about 1.0 wt.%
b. zinc citrate in an amount of about 0.5 wt.%
c. stannous fluoride in an amount of preferably about 0.45 wt.%
d. stannous pyrophosphate in an amount of about 1.0 wt.% and
e. a humectant system comprising glycerin and sorbitol, wherein the sorbitol is present in an amount of about 3.5 wt.%;
or wherein the composition comprises:
f. zinc oxide in an amount of about 1.0 wt.%
g. zinc citrate in an amount of about 0.5 wt.%
h. stannous fluoride in an amount of preferably about 0.45 wt.%
i. stannous pyrophosphate in an amount of about 1.0 wt.% and
j. a humectant system comprising glycerin and sorbitol, wherein the sorbitol is present in an amount of about 5 wt.%,
wherein the wt.% is based on the total weight of the composition.

11. A composition of any of claims 1-9, wherein the composition comprises:
a. zinc oxide in an amount of about 1.0 wt.%
b. zinc citrate in an amount of about 0.5 wt.%
c. stannous fluoride in an amount of preferably about 0.45 wt.%
d. stannous pyrophosphate in an amount of about 1.0 wt.% and
e. a humectant system comprising glycerin and sorbitol, wherein the sorbitol is present in an amount of about 21 wt.%; and
f. a thickening system comprising hydroxyethyl cellulose and carrageenan,
wherein the wt.% is based on the total weight of the composition.

12. A composition of any of claims 1-9, wherein the composition comprises:
a. zinc oxide in an amount of about 1.0 wt.%
b. stannous pyrophosphate in an amount of about 1.0 wt.%;
c. stannous fluoride in an amount of preferably about 0.45 wt.%; and
d. a humectant system comprising glycerin and sorbitol, wherein the sorbitol is present in an amount of about 30 wt.%,
wherein the wt.% is based on the total weight of the composition.

13. The composition of claim 10, further comprising a citrate buffer system, wherein the buffer system comprises tri-sodium citrate and citric acid.

14. The composition of any of the preceding claims, wherein the composition is any of the following selected from: a toothpaste, transparent paste, gel, mouth rinse, spray and chewing gum.

15. A composition of any of the preceding claims for use in a method to improve oral health comprising applying an effective amount of said oral composition, wherein the oral care composition is applied to the oral cavity of a subject in need thereof.

## Patentansprüche

1. Mundpflegezusammensetzung, umfassend:
a. eine Zinkquelle, wobei die Zinkquelle Zinkoxid und Zinkcitrat umfasst;
b. eine erste Quelle von Zinn(II);
c. eine zweite Quelle von Zinn(II), wobei die zweite Quelle von Zinn(II) Zinn(II)-Pyrophosphat umfasst;
d. ein Feuchthaltesystem, das Glycerin und Sorbitol umfasst, und wobei das Sorbitol in einer Menge von 2,5 bis 35 Gew.-% vorhanden ist; und
e. ein Verdickungssystem, das ein Verdickungsmittel umfasst, das ausgewählt ist aus Gruppe bestehend aus Carrageen, Hydroxyethylcellulose und wasserlöslichen Salzen von Celluloseethern und Kombinationen davon,
wobei die Gew.-% auf das Gesamtgewicht der Mundpflegezusammensetzung bezogen ist.

2. Mundpflegezusammensetzung nach Anspruch 1, wobei das Verhältnis der Menge an Zinkoxid zu Zinkcitrat 1,5:1 bis 4,5:1 beträgt.

3. Mundpflegezusammensetzung nach einem der vorstehenden Ansprüche, umfassend Zinkcitrat und Zinkoxid, wobei das Zinkcitrat in einer Menge von 0,25 bis 1,0 Gew.-% vorliegt und das Zinkoxid in einer Menge von 0,75 bis 1,25 Gew.-%, bezogen auf das Gesamtgewicht der Zusammensetzung, vorhanden sein kann.

4. Mundpflegezusammensetzung nach einem beliebigen der vorhergehenden Ansprüche, wobei die erste Zinn(II)-Ionenquelle Zinn(II)-fluorid ist, andere Zinn(II)-halogenide wie Zinn(II)-chlorid-dihydrat, Zinn(II)-pyrophosphat, organische Zinn(II)-carboxylatsalze wie Zinn(II)-formiat, -acetat, -gluconat, -lactat, -tartrat, -oxalat, - malonat und -citrat, Zinn(II)-ethylenglyoxid oder eine Mischung davon.

5. Mundpflegezusammensetzung nach einem beliebigen der vorhergehenden Ansprüche, wobei die erste Zinn(II)-Ionenquelle Zinn(II)-fluorid ist.

6. Mundpflegezusammensetzung nach einem beliebigen der vorhergehenden Ansprüche, wobei die Zusammensetzung ein oder mehrere Alkaliphosphatsalze, ausgewählt aus dibasischem Natriumphosphat, dibasischem Kaliumphosphat, Dicalciumphosphatdihydrat, Calciumpyrophosphat, Tetranatriumpyrophosphat, Tetrakaliumpyrophosphat, Natrium tripolyphosphat, Dinatriumhydrogenorthophosphat, Mononatriumphosphat, Pentakaliumtriphosphat und Mischungen davon umfasst; vorzugsweise wobei die Zusammensetzung ein Alkali-Phosphatsalz in einer Menge von 1 bis 20 Gew.-%, bezogen auf das Gesamtgewicht der Zusammensetzung, umfasst.

7. Mundpflegezusammensetzung nach einem beliebigen der vorhergehenden Ansprüche, wobei das Feuchthaltemittelsystem Glycerin und Sorbitol in einem Gewichtsprozentverhältnis (Glycerin:Sorbitol) von 1:0,10 bis 0,75:1, bezogen auf das Gewicht des Feuchthaltemittelsystems, umfasst.

8. Mundpflegezusammensetzung nach einem beliebigen der vorhergehenden Ansprüche, wobei das Sorbitol in einer Menge von 2,5 bis 25 Gew.-%, bezogen auf das Gesamtgewicht der Zusammensetzung, vorhanden ist.

9. Mundpflegezusammensetzung nach einem beliebigen der Ansprüche 1 bis 8, wobei das Sorbitol in einer Menge von 5 bis 35 Gew.-%, vorzugsweise in einer Menge von 5 bis 25 Gew.-%, oder in einer Menge von 25 bis 35 Gew.-%, bezogen auf das Gesamtgewicht der Zusammensetzung, vorhanden ist.

10. Zusammensetzung nach einem beliebigen der vorhergehenden Ansprüche, wobei die Zusammensetzung umfasst:
a. Zinkoxid in einer Menge von etwa 1,0 Gew.-%
b. Zinkcitrat in einer Menge von etwa 0,5 Gew.-%
c. Zinn(II)-fluorid in einer Menge von vorzugsweise etwa 0,45 Gew.-%
d. Zinn(II)-pyrophosphat in einer Menge von etwa 1,0 Gew.-% und
e. ein Feuchthaltemittelsystem, das Glycerin und Sorbitol umfasst, wobei das Sorbitol in einer Menge von etwa 3,5 Gew.-% vorliegt;
oder wobei die Zusammensetzung umfasst:
f. Zinkoxid in einer Menge von etwa 1,0 Gew.-%
g. Zinkcitrat in einer Menge von etwa 0,5 Gew.-%
h. Zinn(II)-fluorid in einer Menge von vorzugsweise etwa 0,45 Gew.-%
i. Zinn(II)-pyrophosphat in einer Menge von etwa 1,0 Gew.-% und
j. ein Feuchthaltemittelsystem, das Glycerin und Sorbitol umfasst, wobei das Sorbitol in einer Menge von etwa 5 Gew.-% vorliegt,
wobei die Gew.-% auf das Gesamtgewicht der Zusammensetzung bezogen sind.

11. Zusammensetzung nach einem beliebigen der Ansprüche 1 bis 9, wobei die Zusammensetzung umfasst:
a. Zinkoxid in einer Menge von etwa 1,0 Gew.-%
b. Zinkcitrat in einer Menge von etwa 0,5 Gew.-%
c. Zinn(II)-fluorid in einer Menge von vorzugsweise etwa 0,45 Gew.-%
d. Zinn(II)-pyrophosphat in einer Menge von etwa 1,0 Gew.-% und
e. ein Feuchthaltemittelsystem, das Glycerin und Sorbitol umfasst, wobei das Sorbitol in einer Menge von etwa 21 Gew.-% vorliegt; und
f. ein Verdickungssystem, das Hydroxyethylcellulose und Carrageen umfasst,
wobei die Gew.-% auf das Gesamtgewicht der Zusammensetzung bezogen sind.

12. Zusammensetzung nach einem beliebigen der Ansprüche 1 bis 9, wobei die Zusammensetzung umfasst:
a. Zinkoxid in einer Menge von etwa 1,0 Gew.-%
b. Zinn(II)-pyrophosphat in einer Menge von etwa 1,0 Gew.-%;
c. Zinn(II)-fluorid in einer Menge von vorzugsweise etwa 0,45 Gew.-%; und
d. ein Feuchthaltemittelsystem, das Glycerin und Sorbitol umfasst, wobei das Sorbitol in einer Menge von etwa 30 Gew.-% vorliegt,
wobei die Gew.-% auf das Gesamtgewicht der Zusammensetzung bezogen sind.

13. Zusammensetzung nach Anspruch 10, weiterhin umfassend ein Citratpuffersystem, wobei das Puffersystem Trinatriumcitrat und Zitronensäure umfasst.

14. Zusammensetzung nach einem beliebigen der vorhergehenden Ansprüche, wobei die Zusammensetzung aus einem der folgenden ausgewählt ist: einer Zahnpasta, einer transparenten Paste, einem Gel, einer Mundspülung, einem Spray und einem Kaugummi.

15. Zusammensetzung nach einem beliebigen der vorhergehenden Ansprüche zur Verwendung in einem Verfahren zur Verbesserung der Mundgesundheit, umfassend das Auftragen einer wirksamen Menge der Mundzusammensetzung, wobei die Mundpflegezusammensetzung in die Mundhöhle eines Patienten aufgetragen wird, der diese benötigt.

## Revendications

1. Composition de soins bucco-dentaires comprenant :
a. une source de zinc, dans laquelle la source de zinc comprend de l'oxyde de zinc et du citrate de zinc ;
b. une première source de stanneux ;
c. une deuxième source de stanneux, dans laquelle la deuxième source de stanneux comprend du pyrophosphate stanneux ;
d. un système humectant comprenant de la glycérine et du sorbitol, et dans lequel le sorbitol est présent en une quantité de 2,5 à 35 % en poids ; et
e. un système épaississant comprenant un agent épaississant choisi dans le groupe constitué par le carraghénane, l'hydroxyéthylcellulose et les sels solubles dans l'eau d'éthers de cellulose, et les combinaisons de ceux-ci,
dans laquelle le pourcentage en poids est exprimé par rapport au poids total de la composition de soins bucco-dentaires.

2. Composition de soins bucco-dentaires selon la revendication 1, dans laquelle le rapport de la quantité d'oxyde de zinc au citrate de zinc est de 1,5:1 à 4,5:1.

3. Composition de soins bucco-dentaires selon l'une quelconque des revendications précédentes, comprenant du citrate de zinc et de l'oxyde de zinc, dans laquelle le citrate de zinc est présent en une quantité de 0,25 à 1,0 % en poids, et l'oxyde de zinc peut être présent en une quantité de 0,75 à 1,25 % en poids, par rapport au poids total de la composition.

4. Composition de soins bucco-dentaires selon l'une quelconque des revendications précédentes, dans laquelle la première source d'ions stanneux est le fluorure stanneux, d'autres halogénures stanneux tels que le chlorure stanneux dihydraté, le pyrophosphate stanneux, les sels organiques de carboxylate stanneux tels que le formiate, l'acétate, le gluconate, le lactate, le tartrate, l'oxalate, le malonate et le citrate stanneux, le gly-oxyde éthylène stanneux, ou un mélange de ceux-ci.

5. Composition de soins bucco-dentaires selon l'une quelconque des revendications précédentes, dans laquelle la première source d'ions stanneux est le fluorure stanneux.

6. Composition de soins bucco-dentaires selon l'une quelconque des revendications précédentes, dans laquelle la composition comprend un ou plusieurs sels de phosphate alcalin choisis parmi le phosphate de sodium dibasique, le phosphate de potassium dibasique, le phosphate dicalcique dihydraté, le pyrophosphate de calcium, le pyrophosphate tétrasodique, le pyrophosphate tétrapotassique, le tripolyphosphate de sodium, l'hydrogéno-orthophosphate disodique, le phosphate monosodique, le triphosphate pentapotassique et les mélanges de ceux-ci ; de préférence dans laquelle la composition comprend un sel de phosphate alcalin en une quantité de 1 à 20 % en poids, par rapport au poids total de la composition.

7. Composition de soin soins bucco-dentaires selon l'une quelconque des revendications précédentes, dans laquelle le système humectant comprend de la glycérine et du sorbitol selon un rapport en pourcentage en poids (glycérine:sorbitol) de 1:0,10 à 0,75:1, par rapport au poids du système humectant.

8. Composition de soins bucco-dentaires selon l'une quelconque des revendications précédentes, dans laquelle le sorbitol est présent en une quantité de 2,5 à 25 % en poids par rapport au poids total de la composition.

9. Composition de soins bucco-dentaires selon l'une quelconque des revendications 1 à 8, dans laquelle le sorbitol est présent en une quantité de 5 à 35 % en poids, de préférence en une quantité de 5 à 25 % en poids ; ou en une quantité de 25 à 35 % en poids, par rapport au poids total de la composition.

10. Composition selon l'une quelconque des revendications précédentes, dans laquelle la composition comprend :
a. de l'oxyde de zinc en une quantité d'environ 1,0 % en poids
b. du citrate de zinc en une quantité d'environ 0,5 % en poids
c. du fluorure stanneux en une quantité de préférence d'environ 0,45 % en poids
d. du pyrophosphate stanneux en une quantité d'environ 1,0 % en poids, et
e. un système humectant comprenant de la glycérine et du sorbitol, dans lequel le sorbitol est présent en une quantité d'environ 3,5 % en poids ;
ou dans laquelle la composition comprend :
f. de l'oxyde de zinc en une quantité d'environ 1,0 % en poids
g. du citrate de zinc en une quantité d'environ 0,5 % en poids
h. du fluorure stanneux en une quantité de préférence d'environ 0,45 % en poids
i. du pyrophosphate stanneux en une quantité d'environ 1,0 % en poids, et
j. un système humectant comprenant de la glycérine et du sorbitol, dans lequel le sorbitol est présent en une quantité d'environ 5 % en poids,
dans laquelle le % en poids est exprimé par rapport au poids total de la composition.

11. Composition selon l'une quelconque des revendications 1 à 9, dans laquelle la composition comprend :
a. de l'oxyde de zinc en une quantité d'environ 1,0 % en poids
b. du citrate de zinc en une quantité d'environ 0,5 % en poids
c. du fluorure stanneux en une quantité de préférence d'environ 0,45 % en poids
d. du pyrophosphate stanneux en une quantité d'environ 1,0 % en poids, et
e. un système humectant comprenant de la glycérine et du sorbitol, dans lequel le sorbitol est présent en une quantité d'environ 21 % en poids ; et
f. un système épaississant comprenant de l'hydroxyéthylcellulose et du carraghénane,
dans laquelle le pourcentage en poids est exprimé par rapport au poids total de la composition.

12. Composition selon l'une quelconque des revendications 1 à 9, dans laquelle la composition comprend :
a. de l'oxyde de zinc en une quantité d'environ 1,0 % en poids
b. du pyrophosphate stanneux en une quantité d'environ 1,0 % en poids ;
c. du fluorure stanneux en une quantité de préférence d'environ 0,45 % en poids ; et
d. un système humectant comprenant de la glycérine et du sorbitol, dans lequel le sorbitol est présent en une quantité d'environ 30 % en poids,
dans laquelle le pourcentage en poids est exprimé par rapport au poids total de la composition.

13. Composition selon la revendication 10, comprenant en outre un système tampon de citrate, dans laquelle le système tampon comprend du citrate trisodique et de l'acide citrique.

14. Composition selon l'une quelconque des revendications précédentes, dans laquelle la composition est l'une quelconque des compositions suivantes choisie parmi : un dentifrice, une pâte transparente, un gel, un bain de bouche, un spray et une gomme à mâcher.

15. Composition selon l'une quelconque des revendications précédentes pour une utilisation dans un procédé destiné à améliorer la santé bucco-dentaire, comprenant l'application d'une quantité efficace de ladite composition bucco-dentaire, dans laquelle la composition de soins bucco-dentaires est appliquée à la cavité buccale d'un sujet en ayant besoin.
